# EUROPÄISCHE PATENTSCHRIFT

(11) **EP 3 878 759 B1**
(45) Veröffentlichungstag und Bekanntmachung des Hinweises auf die Patenterteilung: **29.03.2023**
(21) Anmeldenummer: 21155397.9
(22) Anmeldetag: 05.02.2021
(51) Int. Cl.: B65B 3/00, A61J 1/05, A61J 1/20, A61M 1/02

(54) **VORRICHTUNG ZUR ABFÜLLUNG VON BLUTPRODUKTEN**
DEVICE FOR FILLING BLOOD PRODUCTS
DISPOSITIF DE REMPLISSAGE DES PRODUITS SANGUINS

(30) Priorität: 10.03.2020 DE 102020106451
(43) Veröffentlichungstag der Anmeldung: 15.09.2021
(73) Patentinhaber: on point medicals GmbH, 9020 Klagenfurt (AT)
(72) Erfinder: TROSCHL, Clemens, 9300 St. Veit an der Glan (AT)
(74) Vertreter: Riebling, Peter

(56) Entgegenhaltungen:
- US-A- 3 566 930
- US-A1- 2003 230 521
- US-A1- 2019 241 287

## Beschreibung

Gegenstand der Erfindung ist eine Vorrichtung zur Abfüllung von Blutprodukten, insbesondere von Serumaugentropfen, nach dem Oberbegriff des Anspruches 1.

Für spezielle Behandlungsmethoden werden Blutprodukte wie z.B. Serum oder plättchenreiches Plasma benötigt. Diese Blutprodukte können autolog oder allogen sein. Autolog bedeutet, dass aus dem eigenen Blut des Patienten diese Produkte gewonnen werden, allogene Blutprodukte hingegen werden von fremden Spendern gewonnen. Insbesondere Serumaugentropfen haben in den letzten Jahren zunehmend an Bedeutung gewonnen, da sie die Heilung der Hornhaut bei gewissen, schweren Augenerkrankungen fördern. Um diese Blutprodukte in kleinere Gefäße wie Augentropfenfläschchen abzufüllen, sind nach GMP-Richtlinien (good manufacturing practice) strengste Reinraumanforderungen notwendig, um Kontaminationen zu vermeiden und Sicherheit zu gewährleisten.

Eine sichere Abfüllung der Blutprodukte außerhalb eines Reinraums erfordert eine geschlossene Vorrichtung, welche leer und steril verpackt an den Anwender (Blutbank oder Spital) geliefert wird. Vor Ort wird dann das Blutprodukt von technisch geschultem Personal in die Vorrichtung gefüllt, verschlossen und für die Patienten in Verabreichungsbehältnisse verpackt.

Derartige geschlossene Vorrichtungen mit Verabreichungsbehältnissen zur Abfüllung von Blutprodukten werden etwa in DE202011004487 U1 oder WO 2014/108852 A1 offenbart.

Diese Vorrichtungen ermöglichen die Herstellung, Lagerung, Transport und Applikation von fertig aliquotierten, direkt am Patienten anwendbaren Eigenserumaugentropfen oder anderer Arzneimittel aus Blut oder Blutprodukten ohne die Notwendigkeit von Reinraumlaboren.

Die verwendeten Verabreichungsbehältnisse sind miteinander über eine Vielzahl von Schläuchen verbunden. Weiters wird jedes Verabreichungsbehältnis mit einem Tropfenauslass versehen. Dadurch ist die Produktion dieser Vorrichtungen außerordentlich zeit- und kostenintensiv, denn sowohl Schläuche als auch Tropfenauslässe werden manuell verklebt.

Bei beiden Vorrichtungen muss jedes Verabreichungsbehältnis einzeln abgetrennt werden, wodurch die Herstellung des Blutproduktes ebenfalls zeitintensiv ist.

Die US 3 566 930 A zeigt eine Vorrichtung zur Abfüllung von Blutprodukten in mindestens zwei Behältnisse, umfassend einen Vorratsbehälter zur Abgabe des Blutproduktes sowie einen damit verbundenen Zuleitungskanal, der flüssigkeitsleitend über jeweils einen Schlauch mit jeweils einem Behältnis verbunden ist. Zusätzlich weisen die Behältnisse eine Entlüftungsöffnung im Anschlussbereich des Schlauches auf, sodass überschüssige Luft in die Umgebung entweichen kann.

Somit kann zwar die in der Vorrichtung vorhandene Luft entweichen, jedoch besteht damit auch das Risiko, dass die Umgebung oder gar das Blutprodukt selbst verunreinigt wird. Zudem dauert die Befüllung relativ lange, da die Behältnisse in Reihe angeordnet sind und erst wenn das erste Behältnis vollständig gefüllt ist das sich aufstauende Blutprodukt das folgende Behältnis vollständig füllen kann.

Der hier vorliegenden Erfindung liegt deshalb die Aufgabe zu Grunde, eine Vorrichtung der eingangs genannten Art so weiter zu bilden, dass die Herstellung der Vorrichtung ökonomischer ist. Weiters soll die Erfindung die Dauer der Befüllung von einer Vielzahl an Verabreichungsbehältnissen mit einem bestimmten Blutprodukt radikal verkürzen und die Arbeit der Labormitarbeiter wesentlich erleichtern. Weiters sollen Patienten von einem Behältnis profitieren, welches speziell für die Anwendung am Auge konzipiert wurde (im Falle der Abfüllung von Serumaugentropfen).

Die Aufgabe wird erfindungsgemäß durch die Merkmale des unabhängigen Patentanspruches gelöst, während vorteilhafte Ausgestaltungen und Weiterbildungen der Erfindung den Unteransprüchen entnommen werden können.

Vorteilhaftes Merkmal ist, dass die Vorrichtung ein plattenförmiges Bauteil umfasst, in dem die Kanäle eines Kanalsystems verlaufen, durch welche die Verabreichungsbehältnisse parallel mit gleicher Menge des Blutproduktes befüllbar sind.

Die vorliegende Erfindung betrifft somit eine Vorrichtung zur gleichmäßigen Verteilung und Abfüllung von Blutprodukten (z.B. autologe Serumaugentropfen oder plättchenreiches Plasma) in kleinere Gefäße, bevorzugt Verabreichungsbehältnisse. Diese Verabreichungsbehältnisse haben beispielsweise ein Fassungsvermögen von 1,5 ml oder 5 ml.

Die Vorrichtung besteht aus einem plattenförmigen Bauteil, welches über Anschlüsse für die Verabreichungsbehältnisse, einen Anschluss (Eingang) für das flüssige Blutprodukt und einen Anschluss (Ausgang) zur Entlüftung verfügt. Ein solches plattenförmiges Bauteil kann beispielsweise die Form einer Scheibe, einer rechteckigen Platte oder eines flachen Polyeders aufweisen, wobei mit der vorliegenden Erfindung sämtliche flache Formgebungen beansprucht werden.

Alle Anschlussstutzen für die Verabreichungsbehältnisse an der Unterseite des plattenförmigen Bauteils sind mit einem Zuleitungskanal und einem Entlüftungskanal versorgt. Das plattenförmige Bauteil kennzeichnet sich dadurch aus, dass die Zuleitungskanäle im Inneren des Bauteils so angeordnet sind, dass alle Behältnisse gleichzeitig parallel und gleichmäßig befüllt werden können.

Mit der Erfindung wird somit eine Vorrichtung zur Abfüllung von Blutprodukten geschaffen, die eine Befüllung der Verabreichungsbehältnisse ermöglicht, bei der die Befüllung aller Verabreichungsbehältnisse gleichzeitig und gleichmäßig erfolgt.

Das Bauteil besitzt eine plattenähnliche Form und besteht entweder aus einer einzelnen Grundplatte oder aus einer Grundplatte die mit mindestens einer Deckplatte kombiniert ist, wobei die mindestens eine Deckplatte fest mit der Grundplatte verbunden ist. Dies wird beispielsweise durch eine Bondingtechnik ermöglicht, wobei durch Hitze und Druck die Platten miteinander verpresst werden. Die Deckplatte kann auch in Form einer Folie ausgeführt sein.

Die Grundplatte - in alleiniger Ausführung oder mit Kombination mindestens einer Deckplatte - verfügt auf der Unterseite über eine Vielzahl von Anschlussstutzen für die Verabreichungsbehältnisse. Die Anschlussstutzen sind für die einfachere Montage der Verabreichungsbehältnisse konisch ausgebildet. Sie besitzen Schneidkanten, um eine stabile und dichte Verbindung zu gewährleisten. Die Verabreichungsbehältnisse können zum Beispiel kommerziell erhältliche Applikatoren für Augentropfen sein. Die Vorrichtung kann ihre Funktion nur dann erfüllen, wenn sämtliche Verabreichungsbehältnisse angeschlossen sind und das plattenförmige Bauteil waagrecht ausgerichtet ist, mit den Verabreichungsbehältnissen auf der Unterseite.

Das plattenförmige Bauteil besitzt einen Anschluss, um einen Behälter mit dem Blutprodukt über einen Schlauch steril anzuschließen. So kann z.B. mit Hilfe einer Spritze ein definiertes Volumen des Blutproduktes aufgenommen werden und über ein Ventil und mit Hilfe der Kanalführung in dem plattenförmigen Bauteil in die Behältnisse abgefüllt werden. Das Serum wird etwa in einem Beutel bereitgestellt. Um die Abfüllung zu ermöglichen, besitzt das Bauteil einen Ausgang zur Entlüftung, um die in den Kanälen und Behältnissen befindliche Luft entweichen zu lassen. An diesem Ausgang zur Entlüftung wird ein Sterilfilter angebracht, um die GMP Anforderungen zu erfüllen.

Im Inneren des Bauteils verläuft vom Anschluss für das Blutprodukt ein Kanal, welcher sich in weitere Kanäle aufteilt, um sämtliche Verabreichungsbehältnisse, die an den Anschlussstutzen aufgesteckt sind, aufzufüllen. Die Kanäle sind so ausgelegt, dass alle Behältnisse gleichzeitig und gleichmäßig befüllt werden können. Das kann beispielsweise über eine Querschnittsveränderung an den Anschlussstutzen erfolgen. Durch diese Querschnittsveränderung kommt es zu einem erhöhten Druck der Flüssigkeit vor der Querschnittsveränderung und einer Beschleunigung der Flüssigkeit in der Querschnittsveränderung (Düseneffekt). Der erhöhte, gleichmäßige Druck vor allen Anschlussstutzen sorgt für die simultane Befüllung aller Verabreichungsbehältnisse.

Sämtliche Anschlussstutzen verfügen über einen Entlüftungskanal, wobei die Entlüftungskanäle gebündelt zum Ausgang für die Entlüftung geleitet werden, welcher einen Anschluss für einen Filter aufweist. Dieser Filter ist beispielsweise ein handelsüblicher Sterilfilter mit einer Porengröße von 0,22 µm.

Für den Befüllprozess wird beispielweise eine Spritze zwischen dem Serumbeutel und dem plattenförmigen Bauteil in die Zuführungsleitung eingebracht. Mit der Spritze kann dann ein definiertes Volumen von etwa 30 bis 50 ml aufgezogen werden, wobei die Spritze mit Hilfe eines speziellen, handelsüblichen Ventils wie eine Pumpe funktioniert und die Flüssigkeit auf der einen Seite des Ventils ansaugt und dann auf der anderen Seite des Ventils ausstößt.

Eine weitere Ausführungsform besteht in der Verwendung einer Schlauchquetschpumpe in Verbindung mit dem Beutel, um den Zufluss des Blutproduktes zu steuern.

Eine weitere Ausführungsform besteht in der Verwendung einer Presse in Verbindung mit dem Beutel, um den Zufluss des Bluproduktes zu steuern.

Eine weitere Ausführungsform besteht in der Anwendung eines Vakuums, um die Behältnisse zu evakuieren und dann über ein Ventil die Flüssigkeit einströmen zu lassen.

Auch ist eine maschinelle Vorrichtung denkbar, um das Blutprodukt in das plattenförmige Bauteil zu befördern.

Zur Montage werden vor der Anbringung der Verabreichungsbehältnisse an den Anschlussstutzen der Grundplatte die Verabreichungsbehältnisse gemäß der Verteilung der Anschlussstutzen auf einer geraden Unterlage angeordnet. Unterstützend kann man hierfür einen Halterahmen verwenden, um die Behältnisse entsprechend anzuordnen, z.B. in einem Gittermuster.

Die Grundplatte wird dann umgedreht und die Anschlussstutzen in die nach oben geöffneten Verabreichungsbehältnisse, welche in Position der Anschlussstutzen auf der Unterlage ausgerichtet sind, eingesteckt.

In einer anderen Ausführungsform wird zur Montage die Grundplatte umgedreht auf eine Unterlage gelegt und die Verabreichungsbehältnisse, welche mit Hilfe des Halterahmens transportabel ausgebildet sind, auf die Anschlussstutzen gesteckt. Die Montage der Verabreichungsbehältnisse, der Sterilfilter und des Anschlusses geschieht im Reinraum. Das Produkt wird dann verpackt und sterilisiert und kann so an den Anwender (Blutbank oder Spital) ausgeliefert werden.

Nach dem Befüllprozess werden die Verabreichungsbehältnisse am oberen Ende nahe dem Bauteil verschweißt und abgetrennt.

Um die Verabreichungsbehältnisse von den Anschlussstutzen des plattenförmigen Bauteils zu trennen, sind beispielhaft die folgenden Verfahren aufgelistet:
- Trennschweißen, z.B. mit quetschend-schneidenden Werkzeugen wie beispielsweise Zangen
- Thermoschweißen mit Sollrissstelle
- Einfrieren der Enden jenseits der Trennstelle vor der Trennung
- Ultraschallschweißen

Vorteilhaftes Merkmal der Erfindung ist, dass mehrere Verabreichungsbehältnisse mit einem Schweiß- und Trennvorgang abgetrennt werden können.

Geeignete Materialen für das erfindungsgemäße plattenförmige Bauteil der Vorrichtung sind beispielsweise Polyvinylchlorid (PVC), Polyurethan (PU, PUR), Polyethylen (PE), Polypropylen (PP) oder Polystyrol (PS). Das Bauteil wird bevorzugt als Spritzgussteil unter Reinraumatmosphäre gefertigt.

Es zeigen:
- Figur 1:: Seitensicht der Vorrichtung
- Figur 2:: Untersicht auf das plattenförmige Bauteil
- Figur 3:: Draufsicht auf das plattenförmige Bauteil
- Figur 4:: Perspektivische Ansicht der Verabreichungsbehältnisse
- Figur 5:: Schematische Darstellung der Kanalverläufe

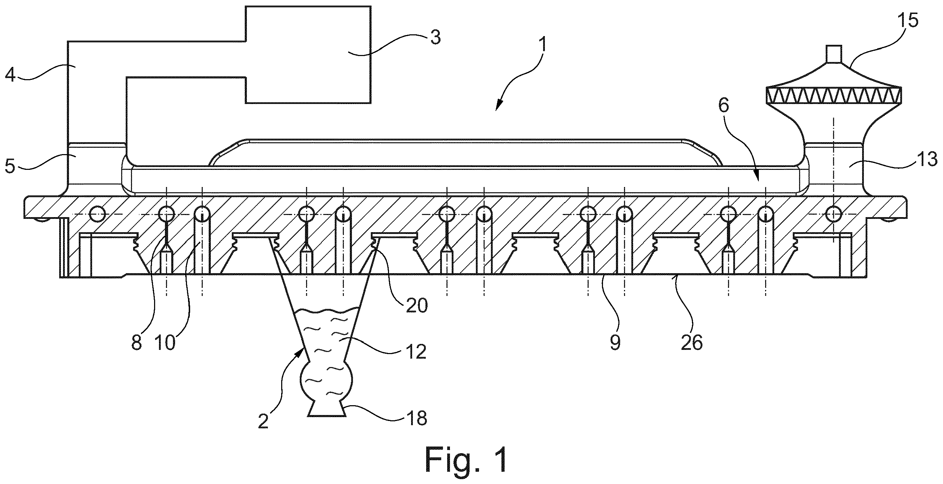

Figur 1 zeigt wie, ausgehend von einem Behälter 3, das Blutprodukt bzw. Blutserum über eine Zuführleitung 4 in das plattenförmige Bauteil 6 eingeleitet wird.

Ein derartiger Behälter 3 kann beispielsweise eine Spritze sein, die an der Zuführleitung 4, welche beispielsweise ein Schlauchstück ist, angebracht ist. Die Zuführleitung 4 ist mit ihrem anderen Ende mit dem Anschluss 5 des plattenförmigen Bauteils 6 verbunden.

So zeigt Figur 1 die Seitenansicht des plattenförmigen Bauteils 6, wobei in diesem Ausführungsbeispiel keine separate Deckplatte das Bauteil von oben her abdeckt und der untere Teil des plattenförmigen Bauteils 6 in Schnittdarstellung dargestellt ist. Das Bauteil 6 weist mehrere Anschlussstutzen 9 auf, welche an der Unterseite 26 des plattenförmigen Bauteils angeformt sind. Ein nicht sichtbarer Zuleitungskanal 7, der horizontal im Inneren des Bauteils 6 verläuft, leitet das über einen Anschluss 5 eingeleitete Blutprodukt in die sichtbaren vertikalen Kanäle 8, welche das Blutprodukt an die einzelnen Anschlussstutzen 9 weiterleiten und gleichmäßig verteilen.

Dazu verläuft der Zuleitungskanal 7 in Längsrichtung des plattenförmigen Bauteils 6 und jeweils mittig zu einem Anschlussstutzen 9 zweigt sich ein Zuleitungskanal 8 ab, der senkrecht von der Zuleitung 7 abgeht. Dieser senkrechte Zuleitungskanal 8 kann eine Querschnittsverengung aufweisen, um eine gleichmäßige Befüllung zu gewährleisten. Gemäß Figur 1 sind fünf Zuleitungskanäle 8 vorhanden, die jeweils ein an einem Anschlussstutzen 9 angebrachtes Verabreichungsbehältnis 2 mit dem flüssigen Blutprodukt 12 befüllen können.

Bevorzugt weist ein Anschlussstutzen 9 eine konische Form auf, um das Aufstecken eines Verabreichungsbehältnisses zu erleichtern. An den Anschlussstutzen 9 können Schneidkanten 20 angebracht sein, um eine stabile und dichte Verbindung mit dem aufgesteckten Verabreichungsbehältnis 2 zu gewährleisten.

Neben einem Zuleitungskanal 8 ist jeder Anschlussstutzen 9 mit einem Entlüftungskanal 10 verbunden, durch den die beim Befüllungsvorgang durch den einfließenden Flüssigkeitsstrom in den Verabreichungsbehältnissen 2 und Kanälen vorhandene Luft entweichen kann. Die Entlüftungskanäle 10 leiten die Luft in den zentralen Entlüftungskanal 11 (nicht sichtbar), der diese wiederum in Richtung des Anschlusses 13 leitet.

Der Anschluss 13 ist luftschlüssig mit dem Filter 15 verbunden, der die Sterilität der gesamten Vorrichtung sicherstellt, während die aus dem plattenförmigen Bauteil 6 strömende Luft durch den zentralen Entlüftungskanal 11 durch den Filter 15 gefiltert an die Umgebung abgegeben werden kann. Bei der gesamten Vorrichtung ist jedoch die Sicherstellung der Sterilität des Blutproduktes vorrangig.

Vorzugsweise ist das Verabreichungsbehältnis 2 mit einem Öffnungsmittel 18 versehen, beispielsweise einer Knickbrechverbindung, welches es dem Verabreichenden ermöglicht, das Verabreichungsbehältnis 2 vor Verwendung auf eine einfache Weise ohne weitere Hilfsmittel zu öffnen.

Durch den Kanal 7 und die davon abzweigenden und damit flüssigkeitsleitend verbundenen Kanäle 8 können alle an den Anschlussstutzen 9 befindlichen Verabreichungsbehältnisse 2 gleichzeitig und vollständig mit dem Blutprodukt 12 gefüllt werden, welches zur Anwendung an den Patienten vorgesehen ist.

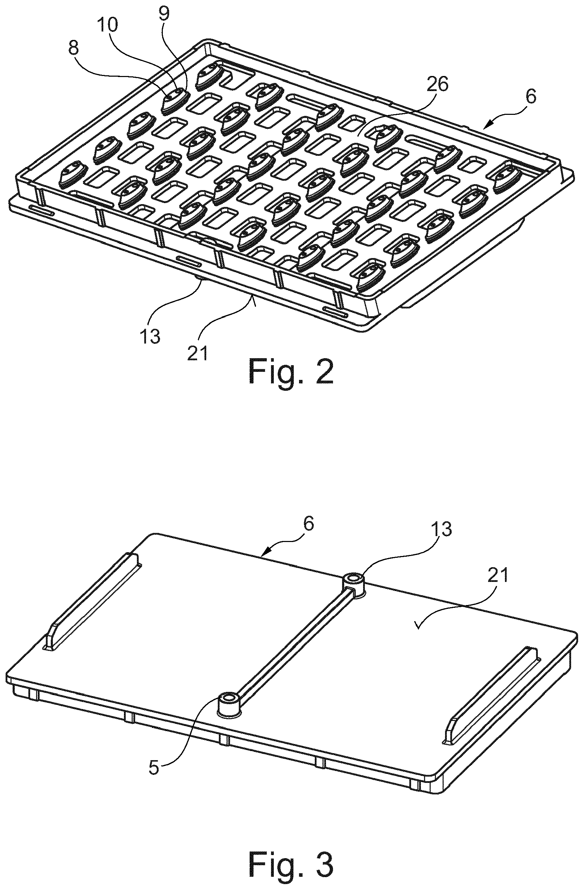

Figur 2 zeigt die Unterseite 26 des plattenförmigen Bauteils 6 mit den einzeln angeordneten Anschlussstutzen 9. Jeder Anschlussstutzen 9 verfügt über einen Zuleitungskanal 8 und einen Entlüftungskanal 10. In dem gezeigten Beispiel nach Figur 2 weist das Bauteil 6 an seiner Unterseite 26 dreißig Anschlussstutzen 9 für den Anschluss von dreißig Verabreichungsbehältnissen 2 auf. Zusätzlich erkennt man einen Teil des Anschlusses 13 an der Oberseite 21 der Platte 6. Des Weiteren sind in dem Baueil 6 viereckige Aussparungen zu erkennen, welche der Gewichtsreduktion dienen. Zudem sind auf beiden Längsseiten Versatz bzw. Führungsschiene angeordnet, mit welcher das plattenförmige Bauteil auf einer Versiegelungs- und Abtrennvorrichtung angebracht wird.

Figur 3 zeigt die Draufsicht auf das Bauteil 6, welches auf seiner Oberseite 21 über einen Anschluss 5, für die Einleitung des Blutprodukts, und einen Anschluss 13, für die Ausleitung der überschüssigen Luft, aufweist, wobei letzterer bevorzugt mit einem Filter in Verbindung steht.

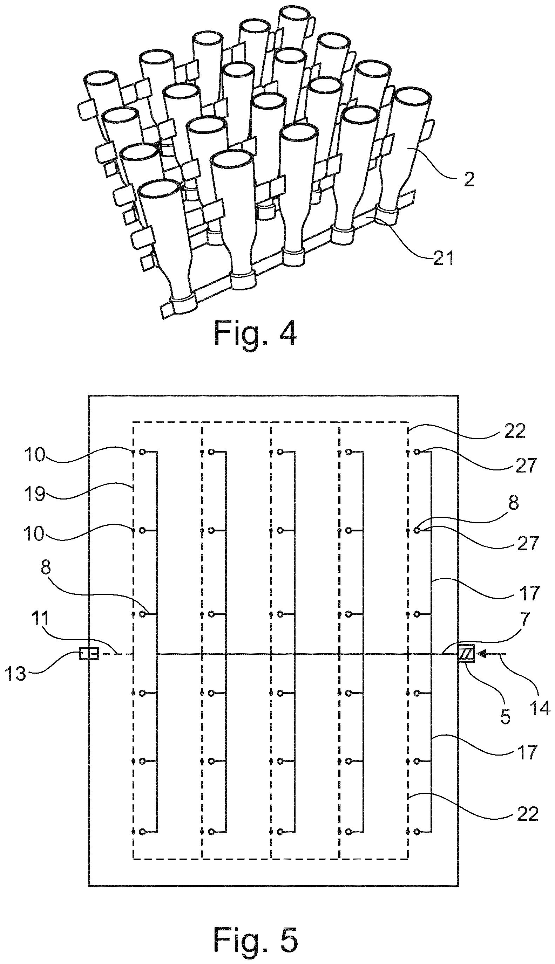

Figur 4 zeigt die Verabreichungsbehältnisse 2, welche bereits entsprechend der Verteilung der Anschlussstutzen 9 auf einer Fläche angeordnet sind. In einem weiteren Verfahrensschritt wird das plattenförmige Bauteil mit seinen Anschlussstutzen auf die Anordnung der Verabreichungsbehältnisse 2 gelegt und die Behältnisse somit mit den Anschlussstutzen 9 verbunden.

Figur 5 zeigt das plattenförmige Bauteil 6 von oben, wobei die Kanalverläufe schematisiert dargestellt sind. Die hier beispielhaft dargestellte Platte enthält dreißig Zuleitungskanäle 8 für die Befüllung der einzelnen Verabreichungsverhältnisse. Jeder Zuleitungskanal 8 ist über einen Verbindungskanal 27 flüssigkeitsleitend mit einem Nebenkanal 17 verbunden, wobei jeder Nebenkanal 17 flüssigkeitsleitend mit dem zentralen Kanal 7 verbunden ist. Der zentrale Kanal 7 wird wiederum über den Anschluss 5 mit dem Blutprodukt befüllt.

Neben jeder Zuleitung 8 ist ein Entlüftungskanal 10 angeordnet, der luftschlüssig mit einem Nebenkanal 22 verbunden ist, wobei die Nebenkanäle 22 wiederum luftschlüssig mit dem Entlüftungskanal 11 verbunden sind. Der Entlüftungskanal wiederum steht in luftschlüssiger Verbindung mit dem Anschluss 13, an dem ein nicht gezeigter Filter angebracht ist.

Um eine vollständige Befüllung aller Verabreichungsbehältnisse 2 zu erreichen, wird eine solche Menge des gewünschten Blutproduktes in die Verabreichungsbehältnisse 2 überführt, so dass der Flüssigkeitsstrom bis in die äußersten Verabreichungsbehältnisse 2 gelangt.

Die Befüllung der Vorrichtung 1 erfolgt aufgrund des hydrostatischen Gefälles selbstständig oder durch Drücken auf den an dem plattenförmigen Bauteil 6 angeschlossenen Behälter 3. Dabei tritt nach Durchströmen der Zuführleitung 4 das Blutprodukt 12 in den Kanal 7 und von dort in die davon abzweigenden Nebenkanäle 17. Von den Nebenkanälen 17 zweigen sich die Verbindungskanäle 17 ab, die an ihrem Ende über die Anschlussstutzen 9 mit den Verabreichungsbehältnissen 2 verbunden sind. Bei steigendem Niveau des Blutproduktes 12 in den Verabreichungsbehältnissen 2 wird die darin befindliche Luft über die Entlüftungskanäle 10 in die Zuleitungskanäle 19 gedrückt und in den Kanal 11 eingeleitet, der mit dem Anschluss 13 in Verbindung steht.

Der Vorgang der Befüllung setzt sich fort, bis das gesamte Blutprodukt aufgebraucht ist und alle angeschlossenen Verabreichungsbehältnisse gefüllt sind. Ggf. wird, um die Behälter besser zu verschweißen, etwas ungefüllten Raum in den Behälter gelassen.

### Zeichnungslegende

- 1: Vorrichtung
- 2: Verabreichungsbehältnis
- 3: Behälter
- 4: Zuführleitung
- 5: Anschluss
- 6: plattenförmiges Bauteil
- 7: Zuleitungskanal (horizontal)
- 8: Zuleitungskanal (vertikal)
- 9: Anschlussstutzen
- 10: Entlüftungskanal
- 11: Entlüftungskanal
- 12: Blutprodukt
- 13: Anschluss
- 14: Pfeilrichtung
- 15: Filter
- 16: (Deckplatte)
- 17: Nebenkanal
- 18: Öffnungsmittel
- 19: Zuleitungskanal
- 20: Schneidkante
- 21: Oberseite
- 22: Nebenkanal
- 26: Unterseite
- 27: Verbindungskanal

## Patentansprüche

1. Vorrichtung (1) zur Abfüllung von Blutprodukten (12) in mindestens zwei Verabreichungsbehältnisse (2), umfassend mindestens einen Behälter (3) zur Abgabe des Blutproduktes (12) innerhalb der Vorrichtung (1), ein Kanalsystem (4, 7, 8, 17), welches flüssigkeitsleitend mit den Verabreichungsbehältnissen (2) verbunden ist und eine Entlüftungsvorrichtung (10, 11, 15), wobei die Vorrichtung (1) ferner ein plattenförmiges Bauteil (6) umfasst, in dem die Kanäle (7, 8, 17) des Kanalsystems verlaufen, durch welche die Verabreichungsbehältnisse (2) parallel mit gleicher Menge des Blutproduktes (12) befüllbar sind, **dadurch gekennzeichnet, dass** durch das plattenförmige Bauteil (6) mehrere Entlüftungskanäle (11) der Entlüftungsvorrichtung verlaufen, die mit den Verabreichungsbehältnissen (2) luftführend verbunden sind.

2. Vorrichtung nach Anspruch 1, **dadurch gekennzeichnet, dass** die Verabreichungsbehältnisse (2) gleichzeitig und gleichmäßig befüllbar sind.

3. Vorrichtung (1) nach Anspruch 1 oder 2, **dadurch gekennzeichnet, dass** die Kanäle (7, 8, 17) in jeweils einem Anschlussstutzen (9) münden, auf denen die Verabreichungsbehältnisse (2) aufgesteckt sind.

4. Vorrichtung (1) nach einem der Ansprüche 1 bis 3, **dadurch gekennzeichnet, dass** die Anschlussstutzen (9) an der Unterseite des plattenförmigen Bauteils (6) angeordnet sind.

5. Vorrichtung (1) nach einem der Ansprüche 1 bis 4, **dadurch gekennzeichnet, dass** jeweils ein Anschlussstutzen (9) mit einem Zuleitungskanal (8) und einem Entlüftungskanal (10) verbunden ist.

6. Vorrichtung (1) nach einem der Ansprüche 1 bis 5, **dadurch gekennzeichnet, dass** in dem plattenförmigen Bauteil (6) ein zentraler Zuleitungskanal (7) das Blutprodukt (12) auf mehrere davon abzweigende Nebenkanäle (17) verteilt, die in die Zuleitungskanäle (8) für die Anschlussstutzen (9) münden.

7. Vorrichtung (1) nach einem der Ansprüche 1 bis 5, **dadurch gekennzeichnet, dass** in dem plattenförmigen Bauteil (6) ein zentraler Zuleitungskanal (7) das Blutprodukt (12) auf mehrere davon abzweigende Nebenkanäle (17) verteilt, von denen mehrere Verbindungskanäle (27) abzweigen, die in die Zuleitungskanäle (8) für die Anschlussstutzen (9) münden.

8. Vorrichtung (1) nach einem der Ansprüche 1 bis 7, **dadurch gekennzeichnet, dass** neben jedem Zuleitungskanal (8) ein Entlüftungskanal (10) angeordnet ist, der luftschlüssig mit dem Anschlussstutzen (9) verbunden ist.

9. Vorrichtung (1) nach einem der Ansprüche 1 bis 8, **dadurch gekennzeichnet, dass** jeder Entlüftungskanal (10) mit einem Zuleitungskanal (19) luftschlüssig in Verbindung steht, welcher wiederum in einem zentralen Entlüftungskanal (11) mündet, der die in den Kanälen und Behältnissen befindliche Luft außerhalb des plattenförmigen Bauteils (6) befördert.

10. Vorrichtung (1) nach einem der Ansprüche 1 bis 9, **dadurch gekennzeichnet, dass** am Ende des zentralen Entlüftungskanals (11) ein Anschluss (13) vorhanden ist, auf dem ein Filter (15) zur Vermeidung jeglicher Kontamination des Blutproduktes (12) angebracht ist.

11. Vorrichtung (1) nach einem der Ansprüche 1 bis 10, **dadurch gekennzeichnet, dass** die Anschlussstutzen (9) konisch ausgebildet sind.

12. Vorrichtung (1) nach einem der Ansprüche 1 bis 11, **dadurch gekennzeichnet, dass** die Zuleitungskanäle (8) eine Querschnittsveränderung i den Anschlussstutzen aufweisen, um die Flüssigkeit unter erhöhten Druck und erhöhter Beschleunigung in die Verabreichungsbehältnisse (2) zu füllen.

## Claims

1. Device (1) for placing blood products (12) in at least two administration containers (2) comprising at least one container (3) for releasing the blood product (12) within the device (1), a channel system (4, 7, 8, 17), which is connected in liquid-conducting manner to the administration containers (2), and a ventilation device (10, 11, 15), wherein the device (1) further comprises a plate-like component (6) in which the channels (7, 8, 17) of the channel system run, through which the administration containers (2) can be filled in parallel with the same quantity of blood product (12), **characterised in that** several ventilation channels (11) of the ventilation device run through the plate-like component (6) and are connected in air-conducting manner to the administration containers (2).

2. Device according to claim 1, **characterised in that** the administration containers (2) can be filled simultaneously and uniformly.

3. Device (1) according to claim 1 or 2, **characterised in that** the channels (7, 8, 17) emerge respectively in a connection (9) to which the administration containers (2) are attached.

4. Device (1) according to one of claims 1 to 3, **characterised in that** the connections (9) are arranged on the underside of the plate-like component (6).

5. Device (1) according to one of claims 1 to 4, **characterised in that** respectively one connection (9) is connected to one supply channel (8) and one ventilation channel (10).

6. Device (1) according to one of claims 1 to 5, **characterised in that** in the plate-like component (6), a central supply channel (7) distributes the blood product (12) to several side-channels (17) branching off therefrom which emerge in the supply channels (8) for the connections (9).

7. Device (1) according to one of claims 1 to 5, **characterised in that** in the plate-like component (6), a central supply channel (7) distributes the blood product (12) to several side-channels (17) branching off therefrom, from which several connecting channels (27) branch off which emerge in the supply channels (8) for the connections (9).

8. Device (1) according to one of claims 1 to 7, **characterised in that** a ventilation channel (10), which is connected in air-tight manner to the connection (9), is arranged next to each supply channel (8).

9. Device (1) according to one of claims 1 to 8, **characterised in that** each ventilation channel (10) is connected in air-tight manner to a supply channel (19) which in turn emerges in a central ventilation channel (11) which conveys the air located in the channels and containers outside of the plate-like component (6).

10. Device (1) according to one of claims 1 to 9, **characterised in that** a connection (13), to which a filter (15) is attached to avoid any contamination of the blood product (12), is present at the end of the central ventilation channel (11).

11. Device (1) according to one of claims 1 to 10, **characterised in that** the connections (9) are configured to be conical.

12. Device (1) according to one of claims 1 to 11, **characterised in that** the supply channels (8) have a change in cross-section at the connections in order to place the liquid in the administration containers (2) under increased pressure and increased acceleration.

## Revendications

1. Dispositif (1) pour mettre des produits sanguins (12) dans au moins deux récipients d'administration (2), comprenant au moins un contenant (3) pour déposer le produit sanguin (12) à l'intérieur du dispositif (1), un système de canaux (4, 7, 8, 17) qui est relié avec transmission de liquide aux récipients d'administration (2) et un dispositif d'évacuation d'air (10, 11, 15), dans lequel le dispositif (1) comprend en outre un composant en forme de plaque (6) dans lequel s'étendent les canaux (7, 8, 17) du système de canaux à travers lesquels les récipients d'administration (2) peuvent être remplis parallèlement avec la même quantité du produit sanguin (12), **caractérisé en ce qu'**à travers le composant en forme de plaque (6) s'étendent plusieurs canaux d'évacuation d'air (11) du dispositif d'évacuation d'air qui sont reliés avec un guidage d'air aux récipients d'administration (2).

2. Dispositif (1) selon la revendication 1, **caractérisé en ce que** les récipients d'administration (2) peuvent être remplis simultanément et uniformément.

3. Dispositif (1) selon la revendication 1 ou 2, **caractérisé en ce que** les canaux (7, 8, 17) débouchent respectivement dans des tubulures de raccordement (9) sur lesquelles les récipients d'administration (2) sont montés.

4. Dispositif (1) selon l'une des revendications 1 à 3, **caractérisé en ce que** les tubulures de raccordement (9) sont disposées du côté intérieur du composant en forme de plaque (6).

5. Dispositif (1) selon l'une des revendications 1 à 4, **caractérisé en ce qu'**une tubulure de raccordement (9) est reliée à chaque fois à un canal d'alimentation (8) et à un canal d'évacuation d'air (10) respectivement.

6. Dispositif (1) selon l'une des revendications 1 à 5, **caractérisé en ce que** dans le composant en forme de plaque (6), un canal d'alimentation central (7) répartit le produit sanguin (12) sur plusieurs canaux secondaires (17) partant dudit canal (7), qui débouchent dans les canaux d'alimentation (8) pour les tubulures de raccordement (9).

7. Dispositif (1) selon l'une des revendications 1 à 5, **caractérisé en ce que** dans le composant en forme de plaque (6), un canal d'alimentation central (7) répartit le produit sanguin (12) sur plusieurs canaux secondaires (17) partant dudit canal (7), d'où partent plusieurs canaux de liaison (27) qui débouchent dans les canaux d'alimentation (8) pour les tubulures de raccordement (9).

8. Dispositif (1) selon l'une des revendications 1 à 7, **caractérisé en ce que** près de chaque canal d'alimentation (8) est disposé un canal d'évacuation d'air (10) qui est relié de manière étanche à l'air à la tubulure de raccordement (9).

9. Dispositif (1) selon l'une des revendications 1 à 8, **caractérisé en ce que** chaque canal d'évacuation d'air (10) est en communication de manière étanche à l'air avec un canal d'alimentation (19) qui débouche lui-même dans un canal d'évacuation d'air central (11) qui transporte à l'extérieur du composant en forme de plaque (6) l'air situé dans les canaux et récipients.

10. Dispositif (1) selon l'une des revendications 1 à 9, **caractérisé en ce qu'**à l'extrémité du canal d'évacuation d'air central (11) est prévu un raccord (13), sur lequel est installé un filtre (15) pour éviter toute contamination du produit sanguin (12).

11. Dispositif (1) selon l'une des revendications 1 à 10, **caractérisé en ce que** les tubulures de raccordement (9) sont agencées de manière conique.

12. Dispositif (1) selon l'une des revendications 1 à 11, **caractérisé en ce que** les canaux d'alimentation (8) présentent un changement de section transversale dans les tubulures de raccordement pour verser le liquide dans les récipients d'administration (2) avec une pression accrue et une accélération accrue.
